# FASCICULE DE BREVET EUROPEEN

(11) **EP 1 215 186 B1**
(45) Date de publication et mention de la délivrance du brevet: **17.09.2014**
(21) Numéro de dépôt: 01403218.9
(22) Date de dépôt: 12.12.2001
(51) Int. Cl.: C04B 41/91, C12Q 1/68, B01J 19/00, G01N 33/543

(54) **Support solide pour l'immobilisation d'oligonucléotides**
Fester Träger für die Immobilisierung von Oligonukleotiden
Solid support for the immobilization of oligonucleotides

(30) Priorité: 14.12.2000 FR 0016314
(43) Date de publication de la demande: 19.06.2002
(73) Titulaire: Commissariat à l'Énergie Atomique et aux Énergies Alternatives, 75015 Paris (FR)
(72) Inventeur: Vinet, Françoise, 38000 Grenoble (FR); Chaton, Patrick, 38570 Theys (FR); Mittler, Frédérique, 38120 Saint Egreve (FR); Barritault, Pierre, 38100 Grenoble (FR)
(74) Mandataire: Audier, Philippe André

(56) Documents cités:
- US-A- 5 919 523

## Description

### Domaine technique

L'invention concerne un support solide pour l'immobilisation d'oligonucléotides. Elle concerne aussi un procédé de réalisation d'un tel support solide.

Le support solide de l'invention est notamment utilisable pour réaliser des dispositifs d'analyse biologique miniaturisés ou biopuces. Ces biopuces selon l'invention peuvent être utilisées par exemple pour le séquençage, pour le criblage (ou "screening" en anglais) du polymorphisme simple de nucléotide (SNP), l'étude de l'expression des gènes, l'identification de microorganismes, l'étude du transcriptome.

### Etat de la technique antérieure

Les biopuces mettent en oeuvre une technique de greffage d'oligonucléotides sur un support solide. Ces oligonucléotides peuvent être obtenus par une réaction de polymérisation en chaîne (PCR) qui fournit des fragments d'ADN de quelques centaines de bases. Ils peuvent être présynthétisés, auquel cas ils comportent entre 6 et 100 mères, ou synthétisés in situ auquel cas ils comportent entre 6 et 60 mères.

La fabrication et l'utilisation de microréseaux (ou "microarrays" en anglais) contenant des sondes biologiques relèvent d'un domaine qui se développe rapidement. Les microréseaux peuvent être fabriqués par synthèse parallèle directement sur un support solide. Ils conduisent dans ce cas à des biopuces de haute densité (plus de 5000 sondes). Les microréseaux peuvent aussi être fabriqués par immobilisation de sondes sur la surface du support solide d'une biopuce. Cette dernière méthode est plus versatile puisqu'elle permet à la fois l'utilisation de produits naturels ou synthétiques qui peuvent être purifiés avant l'immobilisation.

Les supports solides sur lesquels les molécules biologiques sont généralement déposées sont des supports en verre, en silicium, en gel de polyacrylamide, en polymère (voir le brevet américain N° 5 919 523), en plastique (plaques micropuits). Il peut s'agir de membranes en nylon.

Différentes techniques sont utilisables pour déposer les composés biologiques sur les supports en des sites déterminés. Le pipetage permet le dépôt de microgouttes sur les sites : par jet d'encre, piézoélectrique ou par la méthode dite "pin and ring".

Quelle que soit la méthode de dépôt, le support solide doit être traité pour fournir des surfaces d'accrochage ou de greffage pour les molécules biologiques. Ce traitement de surface assure la formation de fonctions chimiques qui sont généralement des fonctions hydroxyles. Ces fonctions- permettent d'assurer les étapes chimiques ultérieures de greffage. De plus, la densité des sites disponibles à cette étape va déterminer la densité finale de sondes biologiques sur le support.

Pour des supports en verre ou en silicium recouvert d'une couche d'oxyde, un nettoyage chimique de la surface permet de lui conférer des groupements hydroxyles, des molécules SiO₂ de surface donnant du SiOH. Le nettoyage peut être réalisé sous des conditions basiques (au moyen de soude ou d'ammoniaque) ou sous des conditions acides (au moyen d'acide chlorhydrique, d'acide sulfochromique ou d'acide sulfo-oxygéné). Des mesures faites par la méthode d'angle de goutte permettent de caractériser l'efficacité de la transformation de l'état de surface du support. L'efficacité du traitement sur l'immobilisation des sondes est caractérisée par hybridation avec des cibles complémentaires. Les observations de fluorescence montrent que, plus la surface du support est hydrophile, meilleure est l'immobilisation des sondes, ce qui permet l'obtention d'une plus grande densité de sondes sur le support.

Cependant, les supports solides de l'art connu présentent une homogénéité de surface pour la fixation des oligonucléotides qui n'est généralement pas assez satisfaisante.

Le document « Photocatalytic activity and photoinduced hydrophilicity of titanium dioxide coated glass » de T. Watanabe et al., Thin Solid Films 351 (1999) 260-263, divulgue un support en verre, supportant une couche de TiO₂ de 300 nm d'épaisseur et dont la surface libre a subi un traitement pour la rendre hydrophile.

### Exposé de l'invention

La présente invention permet de remédier à ce problème de manque d'homogénéité pour la fixation des oligonucléotides.

Un premier objet de l'invention consiste en un support solide présentant une surface convenant à l'immobilisation d'oligonucléotides, caractérisé en ce que ladite surface est la surface d'un matériau choisi parmi HfO₂, Ta₂O₅, ZrO₂ et un mélange comprenant au moins l'un de ces matériaux, ladite surface étant une surface rendue hydrophile par nettoyage par une solution basique ou par une solution acide.

Avantageusement, le matériau se présente sous la forme d'une couche déposée sur un substrat. Cette couche peut posséder une épaisseur comprise entre quelques nanomètres et un micromètre. Le substrat peut être un substrat choisi parmi les substrats en verre, en plastique et en semiconducteur, par exemple en silicium.

Le matériau présentant cette surface d'immobilisation d'oligonucléotides peut être un mélange comprenant SiO₂.

Un deuxième objet de l'invention consiste en une biopuce comprenant un support solide présentant une surface convenant à l'immobilisation d'oligonucléotides, caractérisée en ce que ladite surface est la surface d'un matériau choisi parmi HfO₂, TiO₂, Ta₂O₅, ZrO₂ et un mélange comprenant au moins l'un de ces matériaux, ladite surface étant une surface rendue hydrophile par nettoyage par une solution basique ou par une solution acide.

Un troisième objet de l'invention consiste en un procédé de réalisation d'un support solide présentant une surface convenant à l'immobilisation d'oligonucléotides, le support comprenant un substrat supportant une couche de matériau dont la face libre constitue ladite surface, caractérisé en ce qu'il comporte les étapes suivantes :
- fourniture dudit substrat,
- dépôt sur le substrat d'une couche d'un matériau choisi parmi HfO₂, Ta₂O₅, ZrO₂ et un mélange comprenant au moins l'un de ces matériaux,
- traitement de la face libre de ladite couche pour la rendre hydrophile, l'étape de traitement de la face libre de la couche déposée consistant à nettoyer la couche par une solution basique ou par une solution acide.

L'étape de dépôt peut consister à déposer une couche de matériau d'une épaisseur comprise entre quelques nanomètres et un micromètre.

L'étape de fourniture du substrat peut consister à fournir un substrat choisi parmi les substrats en verre, en plastique et en semiconducteur.

L'étape de dépôt peut consister à déposer un matériau comprenant SiO₂.

L'étape de dépôt peut mettre en oeuvre une méthode de dépôt choisie parmi l'évaporation sous vide, la pulvérisation par faisceau d'ions, la pulvérisation radio-fréquence, la pulvérisation magnétron, le dépôt en phase vapeur de couches atomiques (ALCVD) et le dépôt sol-gel.

Le procédé peut comporter une étape supplémentaire consistant à structurer la face libre de ladite couche. Cette étape de structuration peut mettre en oeuvre une technique choisie parmi la gravure sèche, la gravure humide et le "lift-off".

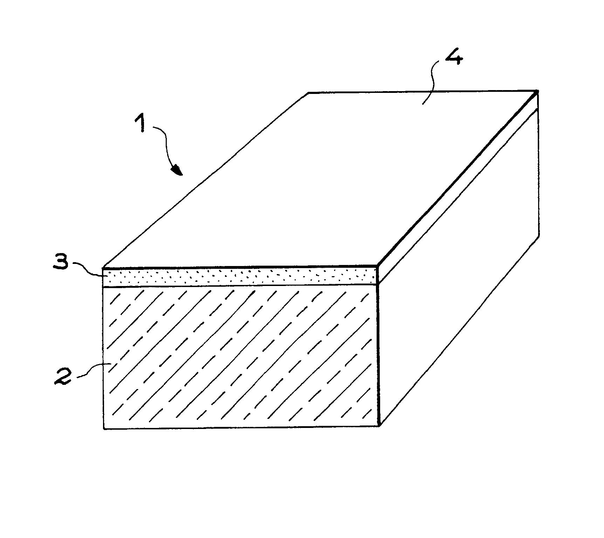

### Brève description du dessin

L'invention sera mieux comprise et d'autres. avantages et particularités apparaîtront à la lecture de la description qui va suivre, donnée à titre d'exemple non limitatif, accompagnée du dessin annexé qui est vue en perspective d'un support solide présentant une surface pour l'immobilisation d'oligonucléotides, selon l'invention.

### Description détaillée d'un mode de réalisation de l'invention

La figure annexée est une vue en perspective d'un support solide 1 selon l'invention. Le support solide 1 comprend un substrat 2 en un matériau permettant le dépôt d'une couche dont la face libre est destinée à constituer une surface pour l'immobilisation d'oligonucléotides. Le substrat 2 est par exemple en silicium.

Une couche 3 est déposée sur le substrat 2. Elle sert de précurseur d'accrochage pour les oligonucléotides. Elle est constituée, en partie ou en totalité, d'un oxyde (ou de plusieurs oxydes) de métal réfractaire TiO₂, ZrO₂, HfO₂ ou Ta₂O₅. La couche déposée est une couche mince d'épaisseur comprise entre quelques nm et 1 µm. Cette couche peut être déposée par évaporation sous vide par canons à électrons à une température comprise entre 50 et 200°C environ. Elle peut être également déposée par pulvérisation par faisceau d'ions (IBS), par pulvérisation radio-fréquence ou magnétron. On peut citer également une technique récente de dépôt : le dépôt en phase vapeur de couches atomiques (ALCVD). On peut citer aussi le dépôt sol-gel.

Les matériaux pouvant constituer la couche mince selon l'invention peuvent être déposés, par les techniques d'évaporation PVD (canons à électrons, IBS, pulvérisation.), indifféremment sur du verre, du plastique, du silicium.

Les oxydes de ces métaux sont très stables vis-à-vis de solutions basiques, ce qui permet une transformation lente et uniforme de la surface.

Sans aucun traitement de surface, ces matériaux sont hydrophobes. Dans le cas de l'oxyde d'hafnium, la mesure d'angle de goutte d'eau conduit à une valeur de 60°. Selon le traitement basique effectué, l'angle de goutte varie de 35° à 6°.

Des supports ainsi traités ont été utilisés pour faire croître des oligonucléotides par synthèse in situ sur un support constitué d'une couche de HfO₂ sur un substrat en silicium. Pour un échantillon de faible hydrophilie (35°), le signal de fluorescence, après hybridation de sondes de 20 mères par des cibles complémentaires, est faible et non uniforme. Un échantillon fortement hydrophile (6°) présente une nette amélioration, principalement en terme d'uniformité.

L'uniformité obtenue a été comparée à celle d'un support solide couramment utilisé à savoir un support formé d'un substrat en silicium recouvert d'une couche d'oxyde thermique de 0,5 µm d'épaisseur. Par rapport à ce support solide de l'art connu, le support selon l'invention (HfO₂ sur Si) procure une amélioration de l'uniformité du signal de fluorescence.

Le même résultat a été obtenu avec un substrat en verre recouvert d'une couche de HfO₂ après immobilisation par liaison covalente de sondes de 20 mères présynthétisées.

Ce type de dépôt peut aussi être utilisé pour des biopuces employant la méthode d'immobilisation de sondes par interactions électrostatiques.

Le dépôt de ces oxydes de métaux réfractaires permet tous les types de greffage utilisés dans les technologies des biopuces, à savoir tout support (verre, silicium, plastique) et tout type de technique de greffage biologique (synthèse in situ, immobilisation par liaison covalente ou liaisons électrostatiques).

Les résultats des mesures montrent une amélioration de l'uniformité des signaux de fluorescence par rapport à l'état de l'art.

## Revendications

1. Support solide (1) présentant une surface (4) convenant à l'immobilisation d'oligonucléotides, **caractérisé en ce que** ladite surface (4) est la surface d'un matériau choisi parmi HfO₂, Ta₂O₅, ZrO₂ et un mélange comprenant au moins l'un de ces matériaux, ladite surface étant une surface rendue hydrophile par nettoyage par une solution basique ou par une solution acide.

2. Support solide selon la revendication 1, **caractérisé en ce que** le matériau se présente sous la forme d'une couche (3) déposée sur un substrat (2).

3. Support solide selon la revendication 2, **caractérisé en ce que** la couche (3) possède une épaisseur comprise entre quelques nanomètres et un micromètre.

4. Support solide selon l'une des revendications 2 ou 3, **caractérisé en ce que** le substrat (2) est un substrat choisi parmi les substrats en verre, en plastique et en semiconducteur.

5. Support solide selon la revendication 4, **caractérisé en ce que** le substrat (2) est en silicium.

6. Support solide selon l'une quelconque des revendications 1 à 5, **caractérisé en ce que** ledit matériau est un mélange comprenant SiO₂.

7. Biopuce comprenant un support solide (1) présentant une surface (4) convenant à l'immobilisation d'oligonucléotides, **caractérisée en ce que** ladite surface (4) est la surface d'un matériau choisi parmi HfO₂, TiO₂, Ta₂O₅, ZrO₂ et un mélange comprenant au moins l'un de ces matériaux, ladite surface étant une surface rendue hydrophile par nettoyage par une solution basique ou par une solution acide.

8. Procédé de réalisation d'un support solide (1) présentant une surface (4) convenant à l'immobilisation d'oligonucléotides, le support (1) comprenant un substrat (2) supportant une couche (3) de matériau dont la face libre constitue ladite surface, **caractérisé en ce qu'**il comporte les étapes suivantes :
- fourniture dudit substrat (2),
- dépôt sur le substrat (2) d'une couche (3) d'un matériau choisi parmi HfO₂, Ta₂O₅, ZrO₂ et un mélange comprenant au moins l'un de ces matériaux,
- traitement de la face libre de ladite couche (3) pour la rendre hydrophile, l'étape de traitement de la face libre de la couche (3) déposée consistant à nettoyer la couche par une solution basique ou par une solution acide.

9. Procédé selon la revendication 8, **caractérisé en ce que** l'étape de dépôt consiste à déposer une couche (3) de matériau d'une épaisseur comprise entre quelques nanomètres et un micromètre.

10. Procédé selon la revendication 8, **caractérisé en ce que** l'étape de fourniture du substrat (2) consiste à fournir un substrat choisi parmi les substrats en verre, en plastique et en semiconducteur.

11. Procédé selon la revendication 8, **caractérisé en ce que** l'étape de dépôt consiste à déposer un matériau comprenant SiO₂.

12. Procédé selon la revendication 8, **caractérisé en ce que** l'étape de dépôt met en oeuvre une méthode de dépôt choisie parmi l'évaporation sous vide, la pulvérisation par faisceau d'ions, la pulvérisation radio-fréquence, la pulvérisation magnétron, le dépôt en phase vapeur de couches atomiques (ALCVD) et le dépôt sol-gel.

13. Procédé selon la revendication 8, **caractérisé en ce qu'**il comporte une étape supplémentaire consistant à structurer la face libre de ladite couche (3).

14. Procédé selon la revendication 13, **caractérisé en ce que** l'étape de structuration met en oeuvre une technique choisie parmi la gravure sèche, la gravure humide et le "lift-off".

## Patentansprüche

1. Fester Träger (1), der eine zur Immobilisierung von Oligonukleotiden geeignete Oberfläche (4) aufweist, **dadurch gekennzeichnet, dass** diese Oberfläche (4) eine Oberfläche aus einem Material ist, das aus HfO₂, Ta₂O₅, ZrO₂ und einem wenigsten eines dieser Materialien umfassenden Gemisch ausgewählt ist, wobei diese Oberfläche eine Oberfläche ist, die durch Reinigen mit einer basischen Lösung oder mit einer sauren Lösung hydrophil gemacht wurde.

2. Fester Träger gemäß Anspruch 1, **dadurch gekennzeichnet, dass** das Material in Form einer auf ein Substrat (2) aufgebrachten Schicht (3) vorliegt.

3. Fester Träger gemäß Anspruch 2, **dadurch gekennzeichnet, dass** die Schicht (3) eine Dicke zwischen einigen Nanometern und einem Mikrometer aufweist.

4. Fester Träger gemäß einem der Ansprüche 2 oder 3, **dadurch gekennzeichnet, dass** das Substrat (2) ein aus Glas-, Kunststoff- und Halbleitersubstraten ausgewähltes Substrat ist.

5. Fester Träger gemäß Anspruch 4, **dadurch gekennzeichnet, dass** das Substrat (2) aus Silicium ist.

6. Fester Träger gemäß einem der Ansprüche 1 bis 5, **dadurch gekennzeichnet, dass** das besagte Material ein SiO₂ umfassendes Gemisch ist.

7. Biochip umfassend einen festen Träger (1), der eine zur Immobilisierung von Oligonukleotiden geeignete Oberfläche (4) aufweist, **dadurch gekennzeichnet, dass** diese Oberfläche (4) eine Oberfläche aus einem Material ist, das aus HfO₂, TiO₂, Ta₂O₅, ZrO₂ und einem wenigsten eines dieser Materialien umfassenden Gemisch ausgewählt ist, wobei diese Oberfläche eine Oberfläche ist, die durch Reinigen mit einer basischen Lösung oder mit einer sauren Lösung hydrophil gemacht wurde.

8. Verfahren zur Herstellung eines festen Trägers (1), der eine zur Immobilisierung von Oligonukleotiden geeignete Oberfläche (4) aufweist, wobei der Träger (1) ein Substrat (2) umfasst, das eine Schicht (3) aus einem Material trägt, dessen freie Fläche die besagte Oberfläche darstellt, **dadurch gekennzeichnet, dass** es die folgenden Schritte umfasst:
- Zuführen des Substrats (2),
- Auftragen einer Schicht (3) aus einem Material, das aus HfO₂, Ta₂O₅, ZrO₂ und einem wenigsten eines dieser Materialien umfassenden Gemisch ausgewählt ist, auf das Substrat (2),
- Behandeln der freien Fläche der Schicht (3), um sie hydrophil zu machen, wobei der Schritt des Behandelns der freien Fläche der aufgetragenen Schicht (3) aus dem Reinigen der Schicht mit einer basischen Lösung oder mit einer sauren Lösung besteht.

9. Verfahren gemäß Anspruch 8, **dadurch gekennzeichnet, dass** der Auftrageschritt aus dem Auftragen einer Schicht (3) aus einem Material mit einer Dicke zwischen einigen Nanometern und einem Mikrometer besteht.

10. Verfahren gemäß Anspruch 8, **dadurch gekennzeichnet, dass** der Schritt des Zuführens des Substrats (2) aus dem Zuführen eines aus Glas-, Kunststoff- und Halbleitersubstraten ausgewählten Substrats besteht.

11. Verfahren gemäß Anspruch 8, **dadurch gekennzeichnet, dass** der Auftrageschritt aus dem Auftragen eines SiO₂ umfassenden Materials besteht.

12. Verfahren gemäß Anspruch 8, **dadurch gekennzeichnet, dass** der Auftrageschritt durch ein Auftrageverfahren bewerkstelligt wird, das aus einer Vakuumverdampfung, lonenstrahlpulverisierung, Radiofrequenzpulverisierung, Magnetronpulverisierung, einem Dampfphasenauftrag atomarer Schichten (ALCVD) und Sol-Gel-Auftrag ausgewählt ist.

13. Verfahren gemäß Anspruch 8, **dadurch gekennzeichnet, dass** es einen zusätzlichen Schritt umfasst, der aus dem Strukturieren der freien Fläche der Schicht (3) besteht.

14. Verfahren gemäß Anspruch 13, **dadurch gekennzeichnet, dass** der Strukturierungsschritt durch eine Technik bewerkstelligt wird, die aus Trockenätzen, Nassätzen und "Lift-off" ausgewählt ist.

## Claims

1. Solid support (1) having a surface (4) for immobilizing oligonucleotides, **characterized in that** said surface (4) is the surface of a material chosen from among HfO₂, TiO₂, Ta₂O₅, ZrO₂ and a mixture containing at least one of these materials, said surface having undergone a treatment to make it hydrophilic by cleaning with a base solution or an acid solution.

2. Solid support according to claim 1, **characterized in that** the material is in the form of a layer (3) deposited on a substrate (2).

3. Solid support according to claim 2, **characterized in that** the layer (3) has a thickness of between a few nanometres and one micrometer.

4. Solid support according to either of claims 2 or 3, **characterized in that** the substrate (2) is a substrate chosen from among substrates in glass, plastic and semiconductor material.

5. Solid support according to claim 4, **characterized in that** the substrate (2) is in silicon.

6. Solid support according to any of claims 1 to 5, **characterized in that** said material is a mixture containing SiO₂.

7. Biochip **characterized in that** it comprises a solid support (1) for immobilizing oligonucleotides, **characterized in that** said surface (4) is the surface of a material chosen from among HfO₂, TiO₂, Ta₂O₅, ZrO₂ and a mixture containing at least one of these materials, said surface having undergone a treatment to make it hydrophilic by cleaning with a base solution or an acid solution.

8. Method for producing a solid support (1) having a surface (4) for immobilizing oligonucleotides, the support (1) comprising a substrate (2) supporting a layer (3) of material whose free face forms said surface, **characterized in that** it comprises the following steps:
- providing said substrate (2),
- depositing on the substrate(2) a layer (3) of a material chosen from among HfO₂, TiO₂, Ta₂O₅, ZrO₂ and a mixture containing at least one of these materials,
- treating the free surface of said layer (3) to make it hydrophilic, the treatment step of the free surface of the deposited layer (3) consisting of cleaning the layer with a base solution or an acid solution.

9. Method according to claim 8, **characterized in that** the deposition step consists of depositing a layer (3) of material having a thickness of between a few nanometres and one micrometer.

10. Method according to claim 8, **characterized in that** the substrate (2) providing step consists of providing a substrate chosen from among glass, plastic and semiconductor substrates.

11. Method according to claim 8, **characterized in that** the deposition step consists of depositing a material containing SiO₂.

12. Method according to claim 8, **characterized in that** the depositing step uses a deposition method chosen from among vacuum evaporation, ion beam sputtering, radio-frequency sputtering, magnetron sputtering, atom layer chemical vapour deposition (ALCVD) and sol-gel deposition.

13. Method according to claim 8, **characterized in that** it comprises an additional step consisting of structuring the free surface of said layer (3).

14. Method according to claim 13, **characterized in that** the structuring step uses a technique chosen from among dry etching, wet etching and "lift-off".
